# EUROPÄISCHE PATENTSCHRIFT

(11) **EP 3 106 111 B1**
(45) Veröffentlichungstag und Bekanntmachung des Hinweises auf die Patenterteilung: **18.04.2018**
(21) Anmeldenummer: 16174443.8
(22) Anmeldetag: 14.06.2016
(51) Int. Cl.: A61B 17/72, A61B 17/70, A61B 17/68

(54) **MECHATRONISCHES IMPLANTAT**
MECHATRONIC IMPLANT
IMPLANT MÉCATRONIQUE

(30) Priorität: 16.06.2015 DE 102015109624
(43) Veröffentlichungstag der Anmeldung: 21.12.2016
(73) Patentinhaber: WITTENSTEIN SE, 97999 Igersheim (DE)
(72) Erfinder: Nill, Eduard, 97078 Würzburg (DE); Friedmann, Jan, 86920 Denklingen (DE)
(74) Vertreter: Zimmermann & Partner Patentanwälte mbB

(56) Entgegenhaltungen:
- EP-A1- 2 422 731
- WO-A1-2010/050890
- US-A1- 2010 114 103
- US-A1- 2010 121 323

## Beschreibung

### Gebiet der Erfindung

Die Erfindung betrifft ein mechatronisches Implantat, insbesondere einen Marknagel.

### Stand der Technik

Aus dem Stand der Technik sind mechatronische Implantate bekannt. Insbesondere sind Implantate bekannt, welche über einen Antrieb verfügen, mit welchem ein erstes Element des Implantats gegenüber einem zweiten Element bewegt werden kann, beispielsweise für die Distraktion eines langen Röhrenknochens.

Aus der EP 2 570 092 ist ein System bekannt, bei welchem zur Verlängerung eines langen Röhrenknochens oder zur Wiederherstellung von verloren gegangenem Knochenmaterial, beispielsweise im Nachgang einer Behandlung eines Tumors, ein Marknagel mit einem Antrieb in den Knochen eingebracht wird. Solche Systeme verlangen eine Steuerung, welche schrittweise die Länge des Marknagels neu einstellt, beispielsweise den Marknagel durch Betätigen des Antriebs jeden Tag jeweils um 1 mm verlängert.

Typische Implantate lassen im Normalbetrieb nur eine Bewegungsrichtung zu. Dies kann je nach Anwendungsfall limitierend sein. Komplexere Systeme, welche beispielsweise über eine Steuerungssoftware im Implantat verfügen könnten hier u.U. Abhilfe schaffen, sind jedoch schwieriger zu überwachen und u.U. nicht ausreichend zuverlässig oder benötigen komplizierte Testverfahren zur Zulassung,

EP 2 422 731 A1 ist der nächstliegende Stand der Technik und beschreibt ein mechatronisches Implantat gemäß Oberbegriff des Anspruchs 1.

### Offenbarung der Erfindung

Aufgabe der Erfindung ist es, ein Implantat oder einen Marknagel anzugeben, welche gegenüber dem Stand der Technik verbessert sind, welche insbesondere eine verbesserte Anwendbarkeit oder einen höheren Komfort für Patienten aufweisen bei möglichst zuverlässiger Funktionsweise.

Die Aufgabe wird mit einem Implantat gemäß Hauptanspruch 1 gelöst.

Ein Aspekt der Erfindung betrifft ein mechatronisches Implantat zur Verwendung in einem menschlichen Körper, mit einem ersten Element, einem zweiten Element, einem Antrieb, der mit dem ersten Element fixiert verbunden ist, wobei der Antrieb einen Abtrieb umfasst, welcher mit dem zweiten Element verbunden ist, um das zweite Element relativ zu dem ersten Element zu bewegen, einem mit dem Antrieb verbunden, implantierbaren Energieempfänger zum drahtlosen Versorgen des Antriebs mit Energie, und mit einem Schalter, um den Antrieb von einer ersten Betriebsrichtung in eine zweite Betriebsrichtung umzuschalten.

Typischerweise wird das zweite Element relativ zu dem ersten Element entlang einer Bewegungsrichtung, beispielsweise linear bewegt. Bei weiteren Ausführungsbeispielen wird das zweite Element rotatorisch oder gemischt linear-rotatorisch relativ zu dem ersten Element bewegt. Eine lineare Bewegung kann beispielsweise entlang einer Längsachse eines distraktionsfähigen orthopädischen Implantates wie beispielsweise einem Marknagel erfolgen.

Der Energieempfänger ist typischerweise zum drahtlosen Versorgen des Antriebs von außerhalb eines Körpers mit Energie eingerichtet. Energie kann bei Ausführungsformen drahtlos beispielsweise über Induktion oder über kapazitive oder mechanische Kopplung übertragen werden.

Typische Ausführungsformen umfassen eine implantierbare Rückmeldeeinrichtung zum Anzeigen, ob der Antrieb in der ersten Betriebsrichtung oder in der zweiten Betriebsrichtung betrieben wird. Typischerweise erfolgt das Anzeigen drahtlos. Dies bietet den Effekt, dass neben einer drahtlosen Energieübertragung auch eine drahtlose Rückmeldung über die Betriebsrichtung möglich ist. Typischerweise erfolgt das Anzeigen nach außerhalb des Körpers. Typische drahtlose Anzeigemittel oder Rückmeldeeinrichtungen zum drahtlosen Anzeigen umfassen Einrichtungen zum Aussenden von Licht, elektromagnetischen Wellen oder von mechanischen Vibrationen.

Typische Ausführungsformen umfassen eine Rückmeldeeinrichtung, welche für eine funkwellenlose Übertragung eingerichtet ist. Dies bietet den Effekt, dass ein Rückkanal der Rückmeldeeinrichtung nicht ebenso wie ein Steuerkanal zum Steuern des mechatronischen Implantats auf Funkwellen zurückgreifen muss. Auf diese Weise kann die Betriebssicherheit erhöht werden.

Typischerweise ist die Rückmeldeeinrichtung bei Ausführungsformen ausgewählt aus: einem akustischen Signalgeber, einem optischen Signalgeber oder einem Vibrations-Signalgeber. Ein akustischer Signalgeber bietet den Effekt, dass er einfach aufgebaut sein kann, ein optischer Signalgeber ist auch bei lauten Umgebungsgeräuschen wahrzunehmen und ein Vibrations-Signalgeber arbeitet unabhängig von Ton oder Licht und kann bei Ausführungsformen auch vom Patienten selbst unmittelbar durch entsprechende subkutane Nerven gespürt werden.

Bei typischen Ausführungsformen ist die Rückmeldeeinrichtung zusammen mit dem Energieempfänger in einem implantierbaren Gehäuse angeordnet. Typischerweise ist die Rückmeldeeinrichtung zusammen mit dem Energieempfänger und zusammen mit dem Schalter in einem implantierbaren Gehäuse angeordnet. Dies bietet einen kompakten Aufbau.

Bei Ausführungsformen wird ein von einem Signalgeber der Rückmeldeeinrichtung ausgegebenes Signal moduliert, beispielsweise wird ein akustisches Signal oder optisches Signal moduliert. Solche Modulationen können beispielsweise eine bestimmte Abfolge von zeitlichen Unterbrechungen des Signals umfassen oder unterschiedliche Tonhöhen oder unterschiedliche Lichtstärken. Auf diese Weise können zusätzliche Informationen beispielweise über eine Geschwindigkeit der Bewegung oder über einen Kraftaufwand oder Energieaufwand, welcher für eine Bewegung notwendig ist, übermittelt werden.

Typischerweise ist die Rückmeldeeinrichtung dazu eingerichtet, eine Information über eine durch den Antrieb bewirkte Bewegungsgeschwindigkeit des zweiten Elements relativ zu dem ersten Element zu übermitteln. Hierfür kann eine Signalmodulation eingesetzt werden, beispielsweise unterschiedliche Tonhöhen, unterschiedliche Lautstärken, unterschiedliche Lichtintensitäten oder Puls-Pause-Verfahren.

Bei typischen Ausführungsformen ist der Schalter ausgewählt aus: einem Reed-Kontakt, einer Photodiode, einem Kondensator und einem elektromechanischen Druckschalter. Bei Verwendung eines Kondensators kann die Haut Dielektrikum verwendet werden. Photodioden reagieren auf Lichtreize von außen, bspw. von extrakorporal, wohingegen elektromechanische Druckschalter durch mechanische Einwirkung umschaltbar sind.

Bei typischen Ausführungsformen umfasst der Antrieb eine elektrische Maschine und/oder ein Getriebe. Elektrische Antriebe bieten den Effekt, dass sie besonders kompakt gebaut sein können und sauber sind. Mit Getrieben lässt sich eine besonders große Kraft bei einem kleinen elektrischen Antrieb erreichen.

Typische Ausführungsformen von mechatronischen Implantaten sind als Marknagel oder als Skoliose-Behandlungseinrichtung ausgebildet. Insbesondere bei Implantaten, welche einer Verschiebung von Knochenteilen relativ zueinander dienen, ist eine Information über die Bewegungsrichtung sinnvoll, da in der Regel nur sehr kleine Bewegungen pro Schritt durchgeführt werden oder durchgeführt werden können, um die Knochen nicht zu überlasten. Bei solchen kleinen Bewegungen ist es nicht auf den ersten Blick ersichtlich, in welche Richtung eine solche Bewegung erfolgt. Bei solchen Anwendungen sind Rückmeldeeinrichtungen zur Information über die Bewegungsrichtung von großem Nutzen.

Typischerweise der Energieempfänger über ein mindestens 10cm-langes Kabel, bei weiteren Ausführungsformen über ein mindestens 15cm-langes oder über ein mindestens 20cm-langes Kabel mit dem Antrieb verbunden. Auf diese Weise kann eine subkutane Implantierung des Energieempfängers, ggf. zusammen mit der Rückmeldeeinrichtung und/oder dem Schalter in einem Gehäuse subkutan implantiert werden, sodass beispielsweise auch eine optische Rückmeldung durch die Haut möglich ist.

### Kurze Beschreibung der Zeichnungen

Weitere Vorteile und Merkmale bevorzugter Ausführungsformen der Erfindung werden nachfolgend anhand der beiliegenden Zeichnungen erläutert, wobei die Figuren zeigen:
- Figur 1: zeigt in einer schematischen Ansicht eine Ausführungsform der Erfindung; und
- Figur 2: zeigt eine Ausführungsform eines Details des Ausführungsbeispiels der Figur 1 in einer weiteren schematischen Ansicht.

### Beschreibung typischer Ausführungsbeispiele

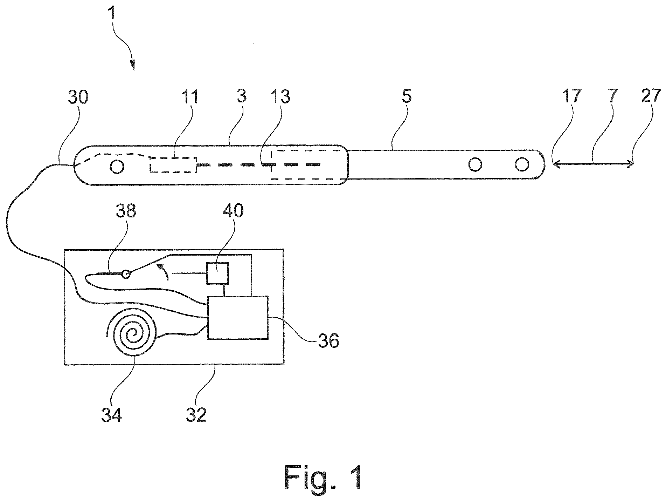

In der Figur 1 ist ein mechatronisches Implantat 1 gezeigt, welches eine typische Ausführungsform darstellt.

Das mechatronische Implantat 1 ist als Marknagel ausgeführt und umfasst ein erstes Element 3 und ein zweites Element 5. Das erste Element 3 ist zumindest teilweise hohl und nimmt das zweite Element 5 teilweise auf, sodass das zweite Element 5 aus dem ersten Element 3 heraus und hinein schiebbar ist entlang einer Bewegungsrichtung 7. Bei dem mechatronischen Implantat 1 der Fig. 1 handelt es sich um einen verstellbaren Marknagel, welcher für Distraktionen von langen Röhrenknochen einsetzbar ist.

Typische Ausführungsformen der Erfindung betreffen verstellbare Marknägel als mechatronische Implantate, weitere Ausführungsformen umfassen Vorrichtungen zur Skoliose-Behandlung oder andere mechatronische Implantate, welche Bewegungen im Körper ausführen können.

Das mechatronische Implantat 1 der Fig. 1 umfasst einen Antrieb 11, welcher eine Spindel als Abtrieb 13 umfasst. Der Abtrieb 13 ist mit dem zweiten Element 5 verbunden. Der Antrieb 11 ist fixiert mit dem ersten Element 3 verbunden bzw. in einem Gehäuse oder einem Hohlzylinder des ersten Elementes 3 aufgenommen.

Durch Betätigen des Antriebs 11 erfolgt eine Bewegung des Abtriebs 13, um das zweite Element 5 entlang der Bewegungsrichtung 7, welche in Längsrichtung des mechatronischen Implantats verläuft, zu bewegen.

Der Antrieb 11 ist dazu eingerichtet, das zweite Element 5 relativ zu dem ersten Element 3 in eine erste Betriebsrichtung 17 und in eine zweite, entgegengesetzte Betriebsrichtung 27 zu bewegen. Die Betriebsrichtungen 17 und 27 verlaufen entlang der Bewegungsachse 7.

Typische Ausführungsformen umfassen einen Antrieb, welcher als elektrische Maschine ausgebildet ist. Weitere Ausführungsformen umfassen hydraulische Antriebe.

Das mechatronische Implantat 1 umfasst weiterhin ein durch ein 20cm langes Kabel 30 mit dem Antrieb verbundenen Energieempfänger 32. Der Energieempfänger 32 umfasst ein Gehäuse, in welchem eine Spule 34 angeordnet ist, welche dazu geeignet ist, durch induktive Energieübertragung Energie zu empfangen.

Das Gehäuse des Energieempfängers 32 kann in einen Körper eines Patienten implantiert werden, typischerweise subkutan. Durch induktive Energieübertragung unter Ausnutzung der Spule 34 ist es möglich, dem Energieempfänger 32 Energie zuzuführen.

Weiterhin verfügt der Energieempfänger 32 über eine Verschaltungseinheit 36, an welcher ein Schalter 38, die Spule 34 und ein Piezo-Summer 40 elektrisch miteinander kontaktiert werden.

Der Schalter 38 kann bei Ausführungsformen als Reed-Kontakt ausgeführt sein, welcher von extra-korporal mittels eines Magneten betätigbar ist. Unter die Begriffe "Reed-Kontakt" und "Schalter" fallen dabei auch Kombinationen mehrerer Reed-Kontakte, beispielsweise zwei Reed-Wechsler-Kontakte zur Umpolung. Durch Betätigung des Schalters 38 kann durch die entsprechende Verschaltung in der Verschaltungseinheit 36 bewirkt werden, dass die Richtung der Bewegung entlang der Bewegungsachse 7 geändert wird, beispielsweise von der ersten Bewegungsrichtung 17 in die zweite Bewegungsrichtung 27 bei Schließen des Schalters und von der zweiten Bewegungsrichtung 27 zu der ersten Bewegungsrichtung 17 bei einem Öffnen des Schalters 38.

Bei typischen Ausführungsformen umfasst der Energieempfänger 32 innerhalb seines Gehäuses den Piezo-Summer 40, welcher betätigt wird, falls der Schalter 38 geschlossen ist und falls Strom durch den Schalter 38 fließt, beispielsweise bei Beaufschlagung der Spule 34 mit elektromagnetischer Energie.

Typischerweise ist die Verschaltungseinheit rein Hardware-mäßig aufgebaut, ist also Software-los. Dies bietet den Vorteil einer hohen Zuverlässigkeit eines einfachen Aufbaus und speziell bei einer Anwendung im medizinischen Bereich auch eventuell vereinfachte Prüfverfahren zur Überprüfung der technischen Zuverlässigkeit des mechatronischen Implantats.

Weitere Ausführungsformen umfassen eine zentrale Steuereinheit der Verschaltungseinheit, wobei die zentrale Steuereinheit Software-basiert arbeiten kann. Dies bietet den Vorteil, dass Neuprogrammierungen vorgenommen werden können.

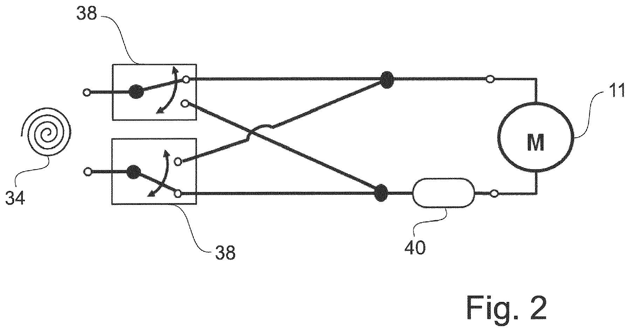

Bei weiteren Ausführungsformen sind andere Schalter als der Reed-Kontakt des Schalters 38 oder andere Rückmeldeeinrichtungen als der Piezo-Summer 40 des Ausführungsbeispiels der Fig.1 vorgesehen. Weitere Beispiele sind an anderer Stelle in dieser Anmeldung genannt. Grundsätzlich ist die Darstellung der Fig. 1 insbesondere bezüglich des Schalters 38 sehr schematisch. Eine genauere Darstellung einer Ausführungsform wird nachfolgend im Zusammenhang mit der Fig. 2 beschrieben.

In der Fig. 2 ist eine Möglichkeit der Verschaltung, wie sie durch die Verschaltungseinheit realisiert werden kann und wie sie bei typischen Ausführungsformen verwendet wird, gezeigt. Dabei werden die bereits im Zusammenhang mit der Fig. 1 beschriebenen Teile nicht nochmals einzeln erläutert, vielmehr werden für gleiche Teile Bezugszeichen verwendet.

Die Spule 34 ist mit dem Schalter 38 verbunden, welcher beispielsweise in Form von zwei Reed-Wechsler-Kontakten ausgeführt ist. Damit ist eine Umpolung und damit Laufrichtungsänderung des Antriebs 11 möglich. Hierzu werden beide Reed-Wechsler-Kontakte gleichzeitig betätigt. Durch Betätigung ist somit eine Richtungsänderung zwischen der ersten Bewegungsrichtung 17 und der zweiten Bewegungsrichtung 27 möglich.

Bei Ausführungsformen ist der Schalter mit einer Mehrzahl von Reed-Kontakten versehen. Bei weiteren Ausführungsformen kann eine elektronische Komponente verwendet werden, um eine Umpolung und damit Laufrichtungsänderung des Antriebs zu erreichen.. Eine typische Ausführungsform verwendet lediglich einen Reed-Kontakt, welcher einen Elektronik-Baustein ansteuert, der zwischen Spule und Antrieb geschaltet ist und bei Betätigung des Schalters den Motor umpolt und somit dessen Drehrichtung ändert.

Je nach Betätigung der Reed-Wechsler-Kontakte des Schalters 38 wird ein in Serie mit einem der Reed-Wechsler-Kontakte geschalteter Piezo-Summer 40 als Rückmeldeeinrichtung betätigt oder bestromt. Durch Betätigung des Piezo-Summers 40 ist es möglich bei einer subkutanen Implantierung entsprechende akustische Signale auch extra-korporal wahrzunehmen. Weiterhin kann es bei Ausführungsformen möglich sein, dass der Patient selbst die Vibration spürt und entsprechend Rückmeldung geben kann. Ausführungsformen können einen an eine elektronische Komponente angeschlossenen Piezo-Summer umfassen oder einen Piezo-Summer, welcher mit einer Freilaufdiode versehen ist, um nur bei einer bestimmten Polung ein Signal zu erzeugen.

Die unterschiedliche Laufrichtung der elektrischen Maschine des Antriebs kann bei Ausführungsformen dadurch erreicht werden, dass ein Spannungszwischenkreis mit negativer Polung geschaffen wird, wobei typischerweise ein Piezo-Summer oder eine andere Anzeigeneinrichtung in den negativ gepolten Spannungszwischenkreis oder in den positiv gepolten Spannungszwischenkreis fest angebracht wird. Bei einer Polung mit zwischengeschaltetem Piezo-Summer kann ein Signal des Piezo-Summers extra-korporal gehört, insbesondere mit einem Stethoskop, werden.

Durch den einfachen Aufbau können sich niedrige oder keine Zulassungsbeschränkungen in dem Bereich der Medizintechnik ergeben.

Typischerweise kann ein Reed-Kontakt durch einen extra-korporal herangeführten Magneten geschaltet werden. Von dem Begriff "Reed-Kontakt" sind grundsätzlich auch Reed-Wechsler-Kontakte umfasst, welche sich dadurch auszeichnen können, dass sie eine Umpolung ermöglichen. Bei typischen Ausführungsformen können der Reed-Kontakt und/oder der Piezo-Summer auf einer gemeinsamen Elektronikplatine, insbesondere zusammen mit einer Spule für einen Energieempfang, aufgenommen sein. Dies bietet einen kompakten Aufbau.

Bei Ausführungsformen wird der Piezo-Summer bei beiden Bewegungsrichtungen aktiviert, beispielweise mit unterschiedlicher Taktung und/oder mit unterschiedlicher Tonhöhe und/oder mit unterschiedlicher Tonabfolge.

Eine Möglichkeit stellt eine Taktung des Antriebs dar, beispielsweise ein Betrieb für 2 Sekunden mit Unterbrechungen für 1 Sekunde in die eine Richtung und einen Betrieb jeweils für 1 Sekunde mit Unterbrechungen von 2 Sekunden in die andere Richtung. Auf diese Weise können unterschiedliche Bewegungsrichtungen insbesondere mit dem Stethoskop von außen detektiert werden.

In der vorstehenden Beschreibung wurden typische Ausführungsbeispiele anhand von Figuren erläutert, die Erfindung ist jedoch nicht auf diese Ausführungsbeispiele beschränkt; vielmehr wird der Umfang der Erfindung durch die Ansprüche bestimmt.

## Patentansprüche

1. Mechatronisches Implantat (1) zur Verwendung in einem menschlichen Körper, mit
- einem ersten Element (3),
- einem zweiten Element (5),
- einem Antrieb (11), der mit dem ersten Element (3) fixiert verbunden ist, wobei der Antrieb (11) einen Abtrieb (13) umfasst, welcher mit dem zweiten Element (5) verbunden ist, um das zweite Element (5) relativ zu dem ersten Element (3) zu bewegen,
- einem mit dem Antrieb (11) verbundenen, implantierbaren Energieempfänger (32) zum drahtlosen Versorgen des Antriebs (11) mit Energie,
- einem Schalter (38), um den Antrieb (11) von einer ersten Betriebsrichtung (17) in eine zweite Betriebsrichtung (27) umzuschalten, **dadurch gekennzeichnet, dass** das Implantat (1)
- mit einer implantierbaren Rückmeldeeinrichtung zum Anzeigen, ob der Antrieb (11) in der ersten Betriebsrichtung (17) oder in der zweiten Betriebsrichtung (27) betrieben wird,
- wobei die Rückmeldeeinrichtung für eine funkwellenlose Übertragung eingerichtet ist.

2. Mechatronisches Implantat (1) nach Anspruch 1, wobei die Rückmeldeeinrichtung ausgewählt ist aus: einem akustischen Signalgeber, einem optischen Signalgeber oder einem Vibrationssignalgeber.

3. Mechatronisches Implantat (1) nach einem der vorhergehenden Ansprüche, wobei die Rückmeldeeinrichtung zusammen mit dem Energieempfänger (32) in einem implantierbaren Gehäuse angeordnet ist.

4. Mechatronisches Implantat (1) nach einem der vorhergehenden Ansprüche, wobei die Rückmeldeeinrichtung dazu eingerichtet ist, eine Information über eine durch den Antrieb (11) bewirkte Bewegungsgeschwindigkeit des zweiten Elementes (5) relativ zu dem ersten Element (3) zu übermitteln

5. Mechatronisches Implantat (1) nach einem der vorhergehenden Ansprüche, wobei der Schalter (38) ausgewählt ist aus: einem Reed-Kontakt, einer Photodiode, einem Kondensator und einem elektromechanischen Druckschalter.

6. Mechatronisches Implantat (1) nach einem der vorhergehenden Ansprüche, wobei der Antrieb (11) eine elektrische Maschine und/oder ein Getriebe umfasst.

7. Mechatronisches Implantat (1) nach einem der vorhergehenden Ansprüche, welches als Marknagel oder als Skoliosebehandlungseinrichtung ausgebildet ist.

8. Mechatronisches Implantat (1) nach einem der vorhergehenden Ansprüche, wobei der Energieempfänger (32) über ein mindestens 20 cm langes Kabel (30) mit dem Antrieb (11) verbunden ist.

## Claims

1. Mechatronic implant (1) for use in the human body, comprising
- a first element (3),
- a second element (5),
- a drive (11) which is fixedly connected to the first element (3), the drive (11) comprising a driven element (13) which is connected to the second element (5) in order to move the second element (5) relative to the first element (3),
- an implantable energy receiver (32) which is connected to the drive (11) and is intended for wirelessly supplying energy to the drive (11),
- a switch (38) for switching the operating direction of the drive (11) from a first operating direction (17) to a second operating direction (27), **characterised in that** the implant (1)
- comprises an implantable feedback device for indicating whether the drive (11) is being operated in the first operating direction (17) or in the second operating direction (27),
- the feedback device being designed for transmission not involving radio waves.

2. Mechatronic implant (1) according to claim 1, wherein the feedback device is selected from: an acoustic signal transmitter, an optical signal transmitter or a vibration signal transmitter.

3. Mechatronic implant (1) according to either of the preceding claims, wherein the feedback device is arranged together with the energy receiver (32) in an implantable housing.

4. Mechatronic implant (1) according to any of the preceding claims, wherein the feedback device is designed to transmit information regarding the speed of a movement of the second element (5) relative to the first element (3) that is brought about by the drive (11).

5. Mechatronic implant (1) according to any of the preceding claims, wherein the switch (38) is selected from: a reed contact, a photodiode, a capacitor and an electromagnetic pressure switch.

6. Mechatronic implant (1) according to any of the preceding claims, wherein the drive (11) comprises an electric machine and/or a gear mechanism.

7. Mechatronic implant (1) according to any of the preceding claims, designed as an intramedullary rod or a scoliosis treatment device.

8. Mechatronic implant (1) according to any of the preceding claims, wherein the energy receiver (32) is connected to the drive (11) by means of a cable (30) that is at least 20 cm in length.

## Revendications

1. Implant mécatronique (1) destiné à être utilisé dans un corps humain, avec
- un premier élément (3),
- un deuxième élément (5),
- un entraînement (11) qui est connecté de manière fixe au premier élément (3), l'entraînement (11) comprenant une sortie (13) qui est connectée au deuxième élément (5) pour déplacer le deuxième élément (5) par rapport au premier élément (3),
- un récepteur d'énergie implantable (32) connecté à l'entraînement (11) destiné à alimenter sans fil l'entraînement (11) en énergie;
- un commutateur (38) destiné à commuter l'entraînement (11) d'une première direction de fonctionnement (17) à une deuxième direction de fonctionnement (27),
**caractérisé par le fait que** l'implant (1) est pourvu
- d'un dispositif de rétroaction implantable destiné à indiquer si l'entraînement (11) est actionné dans la première direction de fonctionnement (17) ou dans la deuxième direction de fonctionnement (27),
- dans lequel le dispositif de rétroaction est conçu pour une transmission sans ondes radio.

2. Implant mécatronique (1) selon la revendication 1, dans lequel le dispositif de rétroaction est choisi parmi: un capteur de signal acoustique, un capteur de signal optique ou un capteur de signal de vibrant.

3. Implant mécatronique (1) selon l'une des revendications précédentes, dans lequel le dispositif de rétroaction est disposé ensemble avec le récepteur d'énergie (32) dans un boîtier implantable.

4. Implant mécatronique (1) selon l'une des revendications précédentes,
dans lequel le dispositif de rétroaction est conçu pour transmettre une information sur une vitesse de déplacement du deuxième élément (5) provoquée par l'entraînement (11) par rapport au premier élément (3).

5. Implant mécatronique (1) selon l'une des revendications précédentes, dans lequel le commutateur (38) est choisi parmi: un contact Reed, une photodiode, un condensateur et un interrupteur de pression électromécanique.

6. Implant mécatronique (1) selon l'une des revendications précédentes, dans lequel l'entraînement (11) comprend une machine électrique et/ou une transmission.

7. Implant mécatronique (1) selon l'une des revendications précédentes, qui est réalisé sous forme de clou intramédullaire ou comme dispositif de traitement de scoliose.

8. Implant mécatronique (1) selon l'une des revendications précédentes, dans lequel le récepteur d'énergie (32) est connecté à l'entraînement (11) par l'intermédiaire d'un câble (30) d'une longueur d'au moins 20 cm.
